# EUROPÄISCHE PATENTANMELDUNG

(11) **EP 4 124 351 A2**
(43) Veröffentlichungstag der Anmeldung: **01.02.2023**
(21) Anmeldenummer: 22184741.1
(22) Anmeldetag: 13.07.2022
(51) Int. Cl.: A61L 2/10

(54) **UV-STERILISATOR**

(30) Priorität: 26.07.2021 DE 102021119274
(71) Anmelder: Marquardt GmbH, 78604 Rietheim-Weilheim (DE)
(72) Erfinder: Götz, Martin, 78050 Villingen-Schwenningen (DE); Willmann, Hubert, 78199 Bräunlingen (DE); Machado, Ilomar Carvalho, 78056 Villingen-Schwenningen (DE); Surdu, Gabriel, 78532 Tuttlingen (DE)
(74) Vertreter: Staeger & Sperling Partnerschaftsgesellschaft mbB

(57) **Zusammenfassung**

Die Erfindung betrifft einen UV-Sterilisator (1) mit einem Gehäuse (2), welches einen Sterilisationsbereich (3) umgibt, einem Halter (5) für ein zu sterilisierendes Element (6) und zumindest zwei UVC-Lichtquellen (4).

## Beschreibung

Die Erfindung betrifft einen UV-Sterilisator mit einem Gehäuse, welches einen Sterilisationsbereich umgibt, einem Halter für ein zu sterilisierendes Element und zumindest zwei UVC-Lichtquellen.

Eine ultraviolette keimtötende Bestrahlung ist eine Desinfektionsmethode, bei der kurzwelliges ultraviolettes Licht (Ultraviolett C oder UV-C) verwendet wird, um Mikroorganismen abzutöten oder zu deaktivieren, indem Nukleinsäuren zerstört und ihre DNA gestört wird, so dass sie nicht mehr in der Lage sind, lebenswichtige zelluläre Funktionen auszuführen. Die UVC-Bestrahlung wird in einer Vielzahl von Anwendungen eingesetzt, z. B. bei der Lebensmittel-, Luft- und Wasserreinigung.

UV-Sterilisatoren aus dem Stand der Technik können in zirkulierenden Luft- oder Wassersystemen stark genug UVC-Licht erzeugen, um diese für Mikroorganismen wie Bakterien, Viren, Schimmelpilze und andere Krankheitserreger unwirtlich zu machen. Beispielsweise können UV-Sterilisatoren mit einem Filtersystem gekoppelt werden, um Luft und Wasser zu desinfizieren.

Die Anwendung UV-Sterilisatoren zur Desinfektion wird vor allem in der medizinischen Hygiene und in sterilen Arbeitsbereichen eingesetzt. Zunehmend wird es auch zur Entkeimung von Trink- und Abwasser eingesetzt, da die Behälter geschlossen sind und umgewälzt werden können, um eine höhere UV-Exposition zu gewährleisten. Nachteilig daran ist jedoch, dass aufgrund einer gewissen Baugröße eines UV-Sterilisators ein umwälzen nicht mehr ohne weiteres möglich ist und deshalb die Gefahr besteht, dass stellenweise eine Bestrahlung mit dem UVC-Licht nicht möglich ist.

Hierfür wurden UVC-Lampen, bei welchen die UCV-Lichtquelle mit Hilfe eines Reflektors auf einer Oberfläche verteilt wird, oder UVC-Sterilisator-Boxen entwickelt, bei welchen die UVC-Lichtquelle mittels reflektierender Wände auf die Oberfläche eines zu sterilisierenden Elements verteilt wird.

In der Druckschrift WO 2020/229861 A1 ist ein Desinfektionssystem mit einer Desinfektionskammer und einer Desinfektionsvorrichtung offenbart. Die Desinfektionskammer hat ein Innenvolumen, eine Vielzahl von Strahlungsquellen, welche, wenn sie aktiviert sind, eine desinfizierende Strahlung abgeben, mindestens ein Registrierungselement für die Desinfektionskammer und eine Steuerung für die Desinfektionskammer, die derart angeordnet ist, dass sie einen Desinfektionsvorgang über die Aktivierung der Vielzahl von Strahlungsquellen durchführt. Dabei hat eine Desinfektionsablage eine UV-transparente Ablagenstruktur, wobei die UV-transparente Ablagenstruktur eine mehrere Kammern aufweisende Aufnahme umfasst, welche zum Aufnehmen und Ausrichten der zu desinfizierenden Gegenstände ausgebildet ist. Nachteilig an dieser Lösung ist jedoch, dass für eine ausreichende Sterilisation eine Vielzahl von Strahlungsquellen benötigt werden, welche um einen zu desinfizierenden Gegenstand herum angeordnet werden müssen, damit die gesamte Oberfläche des Gegenstands bestrahlt wird. Aufgrund der Vielzahl von Strahlungsquellen benötigt dieses Desinfektionssystem jedoch viel Bauraum und ist für einen privaten Gebrauch für unterwegs nicht geeignet.

Die Sterilisation von Alltagsgegenständen, wie beispielsweise einem Handy oder einem Autoschlüssel, hat gerade im Hinblick auf eine Pandemie an Bedeutung gewonnen. Ferner benötigt man einen Sterilisator für den privaten Gebrauch meistens unterwegs.

Eine in den Druckschriften US 9 662 411 B2 und US 10 155 057 B2 offenbarte Sterilisatorvorrichtung kann mobil sein und an verschiedenen Orten eingesetzt werden. Die Sterilisatorvorrichtung verwendet keimtötendes ultraviolettes Licht, um Bakterien und Viren abzutöten. Die verschiedenen darin offenbarten Sterilisationseinheiten und -systeme offenbaren eine verbesserte Verteilung des ultravioletten Lichts und/oder eine verbesserte Fähigkeit zur Ausrichtung und Fokussierung des ultravioletten Lichts auf einen gewünschten Bereich des zu desinfizierenden Gegenstands mittels einer Vielzahl von UV-Lichtquellen, welche beabstandet über die gesamte Innenwandfläche des Gehäuses verteilt sind, und eines geriffelten und reflektierenden Materials, mittels welchem die Innenwandfläche des Gehäuses ausgekleidet ist. Die Sterilisatorvorrichtung kann optional aus nichtmagnetischen Materialien hergestellt sein, so dass die Sterilisationseinheit von MRT-Geräten verwendet werden können, ohne Komplikationen oder Schäden an den MRT-Geräten zu verursachen. Nachteilig an dieser Ausführung ist jedoch, dass die Vielzahl von UV-Glühlampen ebenfalls viel Bauraum benötigen. Darüber hinaus ist ein Reflektor innerhalb der Sterilisatorvorrichtung für eine mobile Anwendung, beispielsweise in einem Kraftfahrzeug, ungeeignet, da bei einem Öffnen des Sterilisators der Fahrer bzw. Fahrgäste oder Passanten bzw. der Gegenverkehr geblendet werden könnten.

Es ist daher Aufgabe der vorliegenden Erfindung, einen UV-Sterilisator bereitzustellen, welcher eine bauraumoptimierte allseitige UVC-Bestrahlung eines zu sterilisierenden Elements verbessert.

Diese Aufgabe wird durch die Merkmalskombination gemäß Patentanspruch 1 gelöst.

Erfindungsgemäß wird ein UV-Sterilisator, insbesondere für einen Innenraum eines Kraftfahrzeugs, mit einem Gehäuse, welches einen Sterilisationsbereich umgibt, einem Halter für ein zu sterilisierendes Element und zumindest zwei UVC-Lichtquellen vorgeschlagen. Die zumindest zwei UVC-Lichtquellen sind dabei an einer Gehäuseinnenwand diametral/diagonal gegenüberliegend angeordnet. Ferner ist der Halter derart in dem Gehäuse ausgebildet, dass das zu sterilisierende Element diametral zu oder zwischen den zumindest zwei UVC-Lichtquellen in dem Sterilisationsbereich anordenbar ist. Außerdem ist vor der jeweiligen UVC-Lichtquelle jeweils ein optisches Element angeordnet. Das jeweilige optische Element ist derart ausgebildet, dass ein von der UVC-Lichtquelle emittiertes UVC-Licht auf dem zu sterilisierenden Element fokussierbar oder streubar ist.

Auf diese Weise ist ein reflektorfreier und bauraumoptimierter UVC-Sterilisator bereitgestellt, welcher für eine Anwendung in Fahrzeuginnenräume geeignet ist. Vorteilhaft daran ist, dass die Optimierung der allseitigen UVC-Bestrahlung des zu sterilisierenden Elements durch die diametrale Anordnung der UVC-Lichtquelle in Kombination mit dem jeweiligen optischen Element gleichzeitig den benötigten Bauraum reduziert. Es erfolgt eine Anordnung des zu sterilisierenden Elements relativ zu der entsprechenden UVC-Lichtquelle, um den Bauraum zu reduzieren und das zu sterilisierende Element gleichmäßig und gleichzeitig von allen Seiten zu bestrahlen. Dabei werden Mikroorganismen, welche sich auf einer Oberfläche des zu sterilisierenden Elements befinden, entweder abgetötet oder deaktiviert. Ferner ist günstig, dass ein Wirkungsgrad zwischen einem von der jeweiligen UVC-Lichtquelle ausgesendeten Licht und dem auf einem zu sterilisierenden Element empfangenen Licht durch das jeweilige optische Element verbessert ist. Darüber hinaus wird eine Bestrahlungszeit des zu sterilisierenden Elements reduziert, da dieses trotz eines geringen Bauraums gleichzeitig von allen Seiten bestrahlt wird. Durch die vollständige Bestrahlung der Fläche und der UVC-Licht Intensität aufgrund des jeweiligen optischen Elements wird der Sterilisationsvorgang verkürzt und optimiert. Außerdem kann eine Leistung der jeweiligen UVC-Lichtquelle in Folge einer Streuung des UVC-Lichts durch das entsprechende optische Element reduziert dimensioniert sein. Ein weiterer Grundgedanke der Erfindung ist, das zu sterilisierende Element in dem Gehäuse des UV-Sterilisators schräg anzuordnen bzw. diametral zu den zumindest beiden UVC-Lichtquellen, damit das seitlich emittierte UVC-Licht die Oberfläche des zu sterilisierenden Elements von oben und von unten erreicht. Das zu sterilisierende Element ist beispielsweise ein Schlüssel, ein Handy, eine Brille oder ein Geldbeutel. Ferner wird als UVC-Licht im Sinne der Erfindung eine UV-Strahlung bzw. elektromagnetische Strahlung im optischen Frequenzbereich mit einer Wellenlänge im Bereich von 280 bis 100 nm betrachtet.

In einer vorteilhaften Ausführungsvariante ist vorgesehen, dass das jeweilige optische Element derart ausgebildet ist, dass das von der UVC-Lichtquelle emittierte UVC-Licht auf das gesamte zu sterilisierenden Element streubar ist. Dadurch ist gewährleistet, dass das gesamte zu sterilisierenden Element sterilisiert wird.

Vorzugsweise ist der UV-Sterilisator derart ausgebildet, dass das optische Element eine Linse, insbesondere eine Fokuslinse oder Streulinse, oder ein Lichtleiter ist. Diese beiden Komponenten sind besonders für eine Streuung bzw. Fokussierung des UVC-Lichts der UVC-Lichtquelle geeignet.

In einem Ausführungsbeispiel der Erfindung ist vorgesehen, dass das optische Element aus einem Silikonmaterial oder einem Quarzglas besteht, wobei das optische Elemente eine Transmission in einem UVC-Bereich von wenigstens 80% aufweist. Dies sind besonders geeignete Materialien für ein optisches Element und auf diese Weise ist gewährleistet, dass das optische Element UVC-lichtdurchlässig ist und der Wirkungsgrad des UV-Sterilisators nicht zu gering ist.

Ferner ist eine Ausführung günstig, bei welcher der Halter aus einem UVC-transparenten Material besteht, insbesondere einem Silikonmaterial oder einem Quarzglas. Dadurch kann eine Fläche des zu sterilisierenden Elements mit dem UVC-Licht bestrahlt werden, obwohl das Element auf dem Halter aufliegt bzw. von diesem gehalten wird.

In einer weiteren vorteilhaften Variante ist erfindungsgemäß vorgesehen, dass der Halter in dem Gehäuse derart ausgebildet ist, dass das zu sterilisierende Element in dem Sterilisationsbereich mit seiner Längserstreckung in einem Winkel von 5° bis 45°, insbesondere in einem Winkel von 10° bis 45° schräg zur Verbindung zwischen den beiden UVC-Lichtquellen, anordenbar ist. Der genaue Winkel ist dabei abhängig von einer Form des zu sterilisierenden Elements und der Größe des Gehäuses des Sterilisators. Unterhalb von 10° ist die UVC-Sterilisationswirkung durch das schräg auftreffende UVC-Licht in seiner Wirkung stark eingeschränkt.

Der erfindungsgemäße UV-Sterilisator ist in eine Ausführungsvariante ausgebildet, dass der Halter durch eine Vielzahl von an einem Gehäuseboden des Gehäuses angeordneten Ablagekufen ausgebildet ist. Es ist beispielsweise jedoch auch eine diametral in dem Gehäuse angeordnete Auflagefläche möglich, Hauptsache der Halter erstreckt sich von dem Gehäuseboden derart, dass das zu sterilisierende Element diametral zu den zumindest zwei UVC-Lichtquellen in dem Sterilisationsbereich anordenbar ist.

In einer bevorzugten Ausführungsform der Erfindung ist das Gehäuse zumindest UVC-Licht-undurchlässig, insbesondere lichtundurchlässig. Vorteilhaft daran ist, dass keine Lichtstrahlen das Gehäuse durchdringen und dementsprechend ein Fahrer bzw. Fahrgäste oder Passanten bzw. der Gegenverkehr nicht geblendet werden können.

In einer alternativen Ausführung des vorliegenden UV-Sterilisators ist ferner vorgesehen, dass das Gehäuse an einem Gehäuseoberteil einen Deckel umfasst, wobei der Deckel ein Rolldeckel oder ein Klappdeckel ist. Dabei ist günstig, dass ein Einlegen und Herausnehmen eines zu sterilisierenden Elements in den Sterilisator einfach gehandhabt werden kann. Durch einen entsprechenden Deckel ist das Gehäuse öffenbar und wiederverschließbar.

Weiter vorteilhaft ist es, wenn das jeweilige optische Element in dem Gehäuse in oder zwischen einer Doppelwand angeordnet ist und die jeweilige UVC-Lichtquelle innerhalb der Doppelwand hinter dem entsprechenden optischen Element angeordnet ist. Dadurch sind sämtliche Komponenten hinter der Doppelwandung vor einer Beschädigung oder einer Verschmutzung geschützt und es ist lediglich der Sterilisationsbereich für das zu sterilisierende Element bei einem geöffneten Gehäuse freigelegt bzw. erreichbar.

In einer vorteilhaften Ausführungsvariante ist vorgesehen, dass die entsprechende UVC-Lichtquelle jeweils auf einer Leiterplatte angeordnet ist. Dadurch kann die UVC-Lichtquelle mit Energie versorgt und gesteuert werden.

Ferner ist eine Ausführung günstig, bei welcher an der jeweiligen Leiterplatte jeweils ein Kühlkörper angeordnet ist. Auf diese Weise ist der UV-Sterilisator bzw. die UVC-Lichtquelle vor einer Überhitzung geschützt.

Vorzugsweise ist der UV-Sterilisator derart ausgebildet, dass das entsprechende optische Element auf der jeweiligen Leiterplatte angeordnet ist. Dabei ist die entsprechende UVC-Lichtquelle zwischen der jeweiligen Leiterplatte und dem entsprechenden optischen Element angeordnet. Vorteilhaft daran ist, dass die Komponenten zu einer kompakten Einheit verbunden sind, wodurch der Bauraum für die entsprechenden Komponenten weiter reduziert ist und die Komponenten besser vor einer Beschädigung geschützt sind.

In einer bevorzugten Ausführungsform ist die UVC-Lichtquelle eine UVC-LED. Dabei ist günstig, dass eine entsprechende LED die für die Sterilisation benötigte Energie im Vergleich zu einer Glühbirne reduziert.

Erfindung gemäß wird ferner ein Kraftfahrzeug mit einem Innenraum vorgeschlagen, in welchem ein UV-Sterilisator gemäß einem der vorhergehenden Ansprüche angeordnet ist. Auf diese Weise ist ein reflektorfreier und bauraumoptimierter UVC-Sterilisator in einem Kraftfahrzeug bereitgestellt.

Die vorstehend offenbarten Merkmale sind beliebig kombinierbar, soweit dies technisch möglich ist und diese nicht im Widerspruch zueinander stehen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Fig. 1: eine schematische Ansicht eines UV-Sterilisators und
- Fig. 2: eine Schnittansicht des UV-Sterilisators.

Die Figuren sind beispielhaft schematisch. Gleiche Bezugszeichen in den Figuren weisen auf gleiche funktionale und/oder strukturelle Merkmale hin.

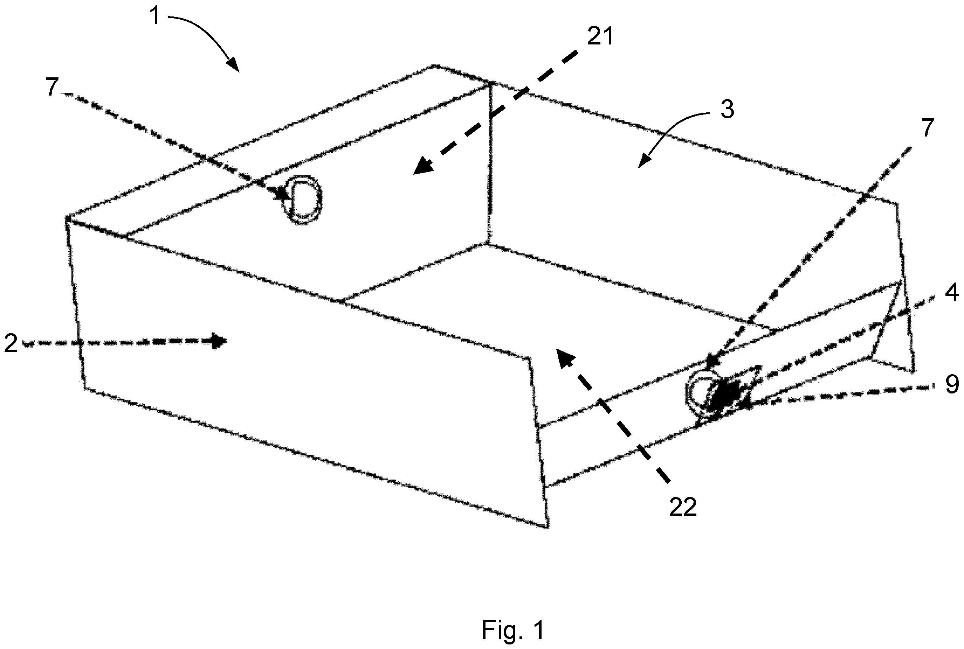

In Figur 1 ist eine schematische Ansicht eines UV-Sterilisators 1 dargestellt. Der UV-Sterilisator 1 ist für einen Innenraum eines Kraftfahrzeugs ausgebildet und umfasst ein Gehäuse 2, welches einen Sterilisationsbereich 3 umgibt. In dem Sterilisationsbereich 3 ist in einem montierten Zustand ein Halter 5 für ein zu sterilisierendes Element 6 angeordnet (nicht dargestellt). Außerdem weist der UV-Sterilisator 1 zwei UVC-Lichtquellen 4 auf, welche diametral gegenüberliegend an einer Gehäuseinnenwand 21 angeordnet sind. Vor der jeweiligen UVC-Lichtquelle 4 befindet sich jeweils ein optisches Element 7, welches in dem Gehäuse 2 in einer Doppelwand angeordnet ist. Dabei ist die jeweilige UVC-Lichtquelle 4 innerhalb der Doppelwand hinter dem entsprechenden optischen Element 7 und die entsprechende UVC-Lichtquelle 4 jeweils auf einer Leiterplatte 9 angeordnet.

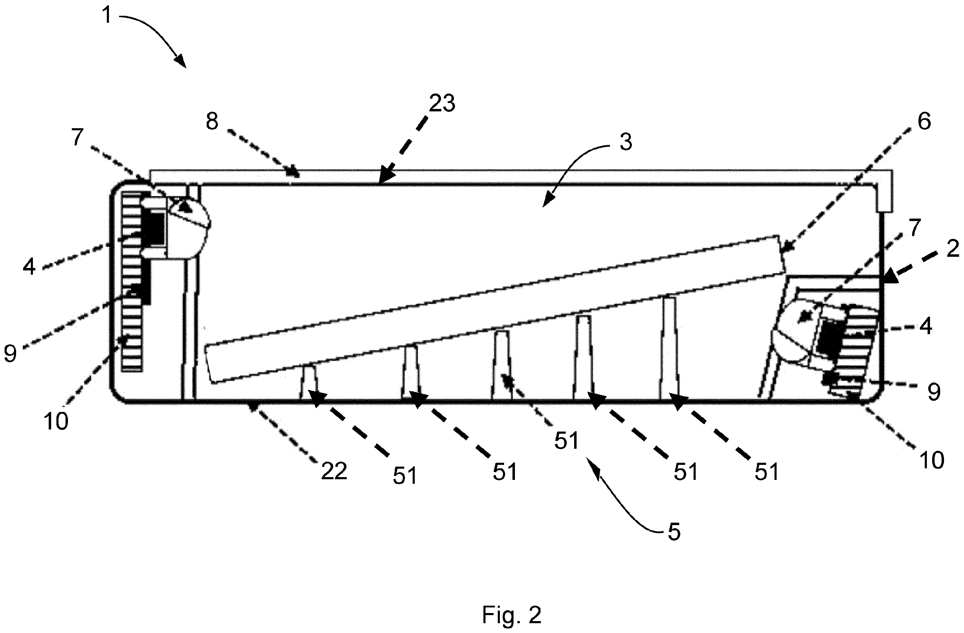

Figur 2 zeigt eine Schnittansicht des in Figur 1 dargestellten UV-Sterilisators 1. Der UV-Sterilisator 1 umfasst ein Gehäuse 2, welches einen Sterilisationsbereich 3 umgibt, einen Halter 5 für ein zu sterilisierendes Element 6 und zwei UVC-Lichtquellen 4.

Das gesamte Gehäuse 2 ist UVC-Licht-undurchlässig, insbesondere Lichtundurchlässig, und umfasst an einem Gehäuseoberteil 23 einen Deckel 8, welcher als ein Rolldeckel ausgebildet ist.

Die zwei UVC-Lichtquellen 4 sind jeweils eine UVC-LED und an einer Gehäuseinnenwand 21 diametral/diagonal gegenüberliegend angeordnet. Zusätzlich zu der UVC-Lichtquelle 4 ist, in dem montierten Zustand der Figur 2, das entsprechende optische Element 7 auf der jeweiligen Leiterplatte 9 sowie die entsprechende UVC-Lichtquelle 4 zwischen der jeweiligen Leiterplatte 9 und dem entsprechenden optischen Element 7 angeordnet.

Das entsprechende vor der jeweiligen UVC-Lichtquelle 4 angeordnete optisches Element 7 ist derart ausgebildet, dass ein von der UVC-Lichtquelle 4 emittiertes UVC-Licht auf dem zu sterilisierenden Element 6 fokussierbar oder streubar ist. Ferner ist das jeweilige optische Element 7 derart ausgebildet, dass das von der UVC-Lichtquelle 4 emittierte UVC-Licht auf das gesamte zu sterilisierenden Element 6 streubar ist. Das jeweilige optische Element 7 ist eine Streulinse, welche aus einem Silikonmaterial oder einem Quarzglas besteht und eine Transmission in einem UVC-Bereich von wenigstens 80% aufweist.

Der Halter 5 ist durch eine Vielzahl von an einem Gehäuseboden 22 des Gehäuses 2 angeordneten Ablagekufen 51 derart in dem Gehäuse 2 ausgebildet, dass das zu sterilisierende Element 6 diametral zu bzw. zwischen den zumindest zwei UVC-Lichtquellen 4 in dem Sterilisationsbereich 3 angeordnet ist. Außerdem besteht der Halter 5 bzw. die jeweilige Ablagekufe 51 aus einem UVC-transparenten Silikonmaterial oder Quarzglas. Ferner ist der Halter 5 bzw. sind die Ablagekufen 51 der Halters 5 in dem Gehäuse 2 derart ausgebildet ist, dass das zu sterilisierende Element 6 in dem Sterilisationsbereich 3 mit seiner Längserstreckung in einem Winkel von 30° zu dem Gehäuseboden 22 angeordnet ist.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. UV-Sterilisator (1) mit einem Gehäuse (2), welches einen Sterilisationsbereich (3) umgibt, einem Halter (5) für ein zu sterilisierendes Element (6) und zumindest zwei UVC-Lichtquellen (4),
wobei die zumindest zwei UVC-Lichtquellen (4) an einer Gehäuseinnenwand (21) diametral/diagonal gegenüberliegend angeordnet sind,
wobei der Halter (5) derart in dem Gehäuse (2) ausgebildet ist, dass das zu sterilisierende Element (6) diametral zu oder zwischen den zumindest zwei UVC-Lichtquellen (4) in dem Sterilisationsbereich (3) anordenbar ist,
wobei vor der jeweiligen UVC-Lichtquelle (4) jeweils ein optisches Element (7) angeordnet ist,
wobei das jeweilige optische Element (7) derart ausgebildet ist, dass ein von der UVC-Lichtquelle (4) emittiertes UVC-Licht auf dem zu sterilisierenden Element (6) fokussierbar oder streubar ist.

2. UV-Sterilisator (1) gemäß Anspruch 1, wobei das jeweilige optische Element (7) derart ausgebildet ist, dass das von der UVC-Lichtquelle (4) emittierte UVC-Licht auf das gesamte zu sterilisierenden Element (6) streubar ist.

3. UV-Sterilisator (1) gemäß Anspruch 1 oder 2, wobei das optische Element (7) eine Linse, insbesondere eine Fokuslinse oder Streulinse, oder ein Lichtleiter ist.

4. UV-Sterilisator (1) gemäß einem der Ansprüche 1 bis 3, wobei das optische Element (7) aus einem Silikonmaterial oder einem Quarzglas besteht, wobei das optische Elemente (7) eine Transmission in einem UVC-Bereich von wenigstens 80% aufweist.

5. UV-Sterilisator (1) gemäß einem der vorhergehenden Ansprüche, wobei der Halter (5) aus einem UVC-transparenten Material besteht, insbesondere einem Silikonmaterial oder einem Quarzglas.

6. UV-Sterilisator (1) gemäß einem der vorhergehenden Ansprüche, wobei der Halter (5) in dem Gehäuse (2) derart ausgebildet ist, dass das zu sterilisierende Element (6) in dem Sterilisationsbereich (3) mit seiner Längserstreckung in einem Winkel von 5° bis 45°, insbesondere in einem Winkel von 10° bis 45° schräg zur Verbindung zwischen den beiden UVC-Lichtquellen (4), anordenbar ist.

7. UV-Sterilisator (1) gemäß einem der vorhergehenden Ansprüche, wobei der Halter (5) durch eine Vielzahl von an einem Gehäuseboden (22) des Gehäuses (2) angeordneten Ablagekufen (51) ausgebildet ist.

8. UV-Sterilisator (1) gemäß einem der vorhergehenden Ansprüche, wobei das Gehäuse (2) zumindest UVC-Licht-undurchlässig ist, insbesondere lichtundurchlässig.

9. UV-Sterilisator (1) gemäß einem der vorhergehenden Ansprüche, wobei das Gehäuse (2) an einem Gehäuseoberteil (23) einen Deckel (8) umfasst, wobei der Deckel (8) ein Rolldeckel oder ein Klappdeckel ist.

10. UV-Sterilisator (1) gemäß einem der vorhergehenden Ansprüche, wobei das jeweilige optische Element (7) in dem Gehäuse (2) in oder zwischen einer Doppelwand angeordnet ist und die jeweilige UVC-Lichtquelle (4) innerhalb der Doppelwand hinter dem entsprechenden optischen Element (7) angeordnet ist.

11. UV-Sterilisator (1) gemäß einem der vorhergehenden Ansprüche, wobei die entsprechende UVC-Lichtquelle (4) jeweils auf einer Leiterplatte (9) angeordnet ist.

12. UV-Sterilisator (1) gemäß dem vorhergehenden Anspruch, wobei an der jeweiligen Leiterplatte (9) jeweils ein Kühlkörper (10) angeordnet ist.

13. UV-Sterilisator (1) gemäß einem der vorhergehenden Ansprüche 11 und 12, wobei das entsprechende optische Element (7) auf der jeweiligen Leiterplatte (9) angeordnet ist, wobei die entsprechende UVC-Lichtquelle (4) zwischen der jeweiligen Leiterplatte (9) und dem entsprechenden optischen Element (7) angeordnet ist.

14. UV-Sterilisator (1) gemäß einem der vorhergehenden Ansprüche, wobei die UVC-Lichtquelle (4) eine UVC-LED ist.

15. Kraftfahrzeug mit einem Innenraum, wobei in dem Innenraum ein UV-Sterilisator (1) gemäß einem der vorhergehenden Ansprüche angeordnet ist.
